(19) Europäisches Patentamt
European Patent Office
Office européen des brevets

(11) EP 1 095 157 B1

(12) EUROPÄISCHE PATENTSCHRIFT

(45) Veröffentlichungstag und Bekanntmachung des Hinweises auf die Patenterteilung:
**08.09.2004 Patentblatt 2004/37**

(21) Anmeldenummer: **99931089.9**

(22) Anmeldetag: **17.06.1999**

(51) Int Cl.⁷: **C12P 19/18**

(86) Internationale Anmeldenummer:
**PCT/EP1999/004199**

(87) Internationale Veröffentlichungsnummer:
**WO 1999/067412 (29.12.1999 Gazette 1999/52)**

(54) **VERFAHREN ZUR HERSTELLUNG WASSENRUNLÖSLICHER alpha-1,4-GLUCANE**

METHOD FOR PRODUCING WATER-INSOLUBLE alpha-1,4-GLUCANS

PROCEDE POUR PRODUIRE DES alpha-1,4-GLUCANES NON SOLUBLES DANS L'EAU

(84) Benannte Vertragsstaaten:
**AT BE CH DE DK ES FR GB GR IE IT LI LU MC NL PT SE**

(30) Priorität: **24.06.1998 DE 19827978**

(43) Veröffentlichungstag der Anmeldung:
**02.05.2001 Patentblatt 2001/18**

(73) Patentinhaber: **Celanese Ventures GmbH
65926 Frankfurt am Main (DE)**

(72) Erfinder:
- **QUANZ, Martin
D-10557 Berlin (DE)**
- **PROVART, Nicholas
D-14163 Berlin (DE)**
- **BANASIAK, Ronald
D-14467 Potsdam (DE)**

(74) Vertreter: **Luderschmidt, Schüler & Partner
Patentanwälte
Industriepark Höchst
65926 Frankfurt (DE)**

(56) Entgegenhaltungen:
**WO-A-95/31553**

- **CHEMICAL ABSTRACTS, vol. 105, no. 21, 24. November 1986 (1986-11-24) Columbus, Ohio, US; abstract no. 187291, RIOU, JEAN YVES ET AL: "Structure of the exocellular D- glucan produced by Neisseria polysaccharea" XP002118633 & CAN. J. MICROBIOL. (1986), 32(12), 909-11 ,**
- **CHEMICAL ABSTRACTS, vol. 91, no. 21, 19. November 1979 (1979-11-19) Columbus, Ohio, US; abstract no. 171287, BIRKHED, D. ET AL: "Structure of extracellular polysaccharides synthesized from sucrose by Neisseria isolated from human dental plaque" XP002118634 & ARCH. ORAL BIOL. (1979), 24(1), 63-6 ,**
- **CHEMICAL ABSTRACTS, vol. 90, no. 7, 12. Februar 1979 (1979-02-12) Columbus, Ohio, US; abstract no. 50049, MACKENZIE, C. R. ET AL: "Structure of the D- glucans produced by Neisseria perflava" XP002118635 & CAN. J. MICROBIOL. (1978), 24(11), 1419-22 ,**
- **CHEMICAL ABSTRACTS, vol. 80, no. 13, 1. April 1974 (1974-04-01) Columbus, Ohio, US; abstract no. 67797, OKADA, GENTARO ET AL: "New studies on amylosucrase, a bacterial.alpha.-D-glucosylase that directly converts sucrose to a glycogen-like.alpha.-glucan" XP002118636 in der Anmeldung erwähnt & J. BIOL. CHEM. (1974), 249(1), 126-35 ,**
- **PETERSEN; SVENSSON; PEDERSEN: 'Carbohydrate Bioengineering, P. 313-320', 1995, ELSEVIER SCIENCE B.V., AMSTERDAM**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein in vitro-Verfahren zur Herstellung wasserunlöslicher α-1,4-Glucane in einem pufferfreien System.

Das Interesse der Industrie an biotechnologischen Verfahren zur Herstellung von Polysacchariden, insbesondere von wasserunlöslichen α-1,4-Glucanen, die auf klassisch organisch synthetischem Wege nicht oder nur sehr schwer zugänglich sind, ist groß. Aus Kostengründen haben es bisher jedoch nur wenige dieser Verfahren bis zur kommerziellen Nutzung gebracht. Gegenüber dem klassischen Weg der organischen Synthesechemie bieten biotechnologische Verfahren Vorteile. So verlaufen enzymatisch katalysierte Reaktionen in der Regel mit viel höheren Spezifitäten (Regiospezifität, Stereospezifität), mit höheren Reaktionsgeschwindigkeiten, unter milderen Reaktionsbedingungen und führen zu höheren Ausbeuten. Diese Faktoren sind bei der Herstellung neuer Polysaccharide von herausragender Bedeutung.

Biotransformationen, d.h. die in vitro-Umsetzung von Substanzen mit gereinigten oder partiell gereinigten Enzymen, bieten im Vergleich zu biotechnologischen in-vivo-Verfahren weitere Vorteile. Sie zeichnen sich gegenüber den in-vivo-Verfahren durch eine bessere Steuerbarkeit und eine höhere Reproduzierbarkeit aus, da die Reaktionsbedingungen in vitro, im Gegensatz zu den Bedingungen in einem lebenden Organismus, definiert eingestellt werden können. Dies ermöglicht die Herstellung von gleichbleibenden Produkten großer Einheitlichkeit und Reinheit und damit von hoher Qualität, die für die weitere industrielle Nutzung von großer Bedeutung ist. Die Aufarbeitung von Produkten gleichbleibender Qualität führt zu Kostensenkungen, weil die Verfahrensparameter, die für die Aufarbeitung erforderlich sind, nicht für jeden Aufarbeitungsansatz neu optimiert werden müssen. Ein weiterer Vorteil von in vitro-Verfahren liegt darin, daß die Produkte im Gegensatz zu in-vivo-Verfahren per se frei von den Organismen sind. Für bestimmte Anwendungen in der Nahrungsmittelindustrie und der Pharmaindustrie ist dies dringend erforderlich. Um die vorteilhaften Eigenschaften wasserunlöslicher α-1,4-Glucane großtechnisch nutzen zu können, besteht ein dringender Bedarf diese kostengünstig zur Verfügung zu stellen. In großem Maßstab sind bisher nur wasserlösliche α-1,4-Glucane, z.B. in Form von Amylose zugänglich. Zur Herstellung von wasserunlöslichen α-1 ,4-Glucanen wurde bisher in der Patentanmeldung WO95/31553 und bei Remaud-Simon et al. (Remaud-Simon, in Petersen, Svenson and Pedersen (Eds.) Carbohydrate bioengineering; Elsevier Science B.V., Amsterdam, The Netherlands (1995), S. 313 - 320) ein Verfahren unter Verwendung einer Amylosaccharase aus Neisseria polysaccharea beschrieben. Dieses in vitro-Verfahren basiert auf der Umsetzung von Saccharose zu α-1,4-Glucanen und Fructose mit einer partiell gereinigten Amylosaccharase und wird in einem Natriumcitrat-Puffer (pH 6,5) bzw. einem Natrium-Maleat-Puffer (pH 6,4) durchgeführt. Folgender Reaktionsmechanismus wurde in der WO 95/31553 postuliert:

$$\text{Saccharose} + (\alpha\text{-1,4-Glucan})_n \rightarrow \text{Fructose} + (\alpha\text{-1,4-Glucan})_{n+1}$$

Ausgehend von diesem Reaktionsschema dienen lineare oligomere oder polymere α-1,4-Glucane als Akzeptoren für eine kettenverlängernde Reaktion, die zu wasserunlöslichen α-1,4-Glucan-Polymeren führt. Im Gegensatz zur WO 95/31553 verwendeten Remaud-Simon et al. (supra) zusätzlich 0,1 g/l Glycogen als exogenen Polysaccharidakzeptor. Dieser verzweigte Polysaccharidakzeptor führte zu einer Erhöhung der Reaktionsgeschwindigkeit im Vergleich zur Biotransformation in Abwesenheit eines exogenen Polysaccharidakzeptors.

Die bisher beschriebenen Systeme zur Herstellung von Polyglucanen unter Verwendung von Amylosaccharasen verlaufen in gepufferten wäßrigen Lösungen. Nicht alle diese Verfahren liefern wasserunlösliche α-1,4-Glucane. Die Verwendung der Pufferchemikalien und die zur Einstellung der erforderlichen Pufferbedingungen benötigte Arbeitszeit führen zu erheblichen Verfahrenskosten und erschweren somit die kommerzielle Nutzung dieser Systeme. Weitere Kosten entstehen durch Reinigungsschritte, die benötigt werden, um Reste der Puffersalze aus den Produkten der Biotransformation (α-1,4-Glucane und Fructose) zu entfernen. Dies ist vor allem bei der Verwendung dieser Produkte in der Nahrungsmittel- und Pharmaindustrie von großer Bedeutung. Es besteht daher ein Bedarf an Verfahren zur effizienten Herstellung von wasserunlöslichen α-1,4-Glucanen, das kommerziell nutzbar ist und zu hochreinen Produkten führt.

Der vorliegenden Erfindung liegt somit die Aufgabe zugrunde, ein für die großtechnische Herstellung von wasserunlöslichen α-1,4-Glucanen geeignetes Verfahren zur Verfügung zu stellen, das zudem zu hochreinen Produkten führt.

Diese Aufgabe wird durch die Bereitstellung der in den Patentansprüchen gekennzeichneten Ausführungsformen gelöst.

Somit betrifft die vorliegende Erfindung ein Verfahren zur Herstellung wasserunlöslicher α-1,4-Glucane, bei dem Saccharose in vitro durch ein Enzym mit der enzymatischen Aktivität einer Amylosaccharase zu wasserunlöslichen α-1,4-Glucanen und Fructose umgesetzt wird, dadurch gekennzeichnet, daß die Umsetzung in einem wäßrigen, pufferfreien System durchgeführt wird.

Es wurde überraschenderweise gefunden, daß zur in vitro-Herstellung von wasserunlöslichen α-1,4-Glucanen durch eine Amylosaccharase aus Neisseria polysaccharea ein wäßriges pufferfreies System verwendet werden kann. Die Effizienz dieses Verfahrens, die anhand der Fructosefreisetzung bzw. des Saccharoseverbrauchs bestimmt werden kann, entspricht der des gepufferten Systems. Dies ist überraschend, weil die Funktionalität verwandter Enzyme bisher nur in gepufferten Lösungen nachgewiesen werden konnte (MacKenzie et al., Can. J. Microbiol. 23 (1977), 1303-1307; Okada und Hehre, J. Biol. Chem. 249 (1974), 126-135; Tao et al., Carbohydrate Res. 181 (1988), 163-174; Büttcher et al, J. Bacteriol. 179 (1997), 3324-3330; WO 95/31553).

Das erfindungsgemäße Verfahren ermöglicht nun eine starke Reduzierung der Kosten der in vitro-Herstellung unlöslicher α-1,4-Glucane. Insbesondere entfallen Arbeitsschritte und Vorrichtungen im Zusammenhang mit der Herstellung von Pufferlösungen sowie mit der Einstellung und gegebenenfalls Aufrechterhaltung des pH-Wertes. Ein weiterer entscheidender Vorteil des erfindungsgemäßen Verfahrens liegt auch im erhöhten Reinheitsgrad der Produkte, der vor allem für Anwendungen im Lebensmittelbereich und in der Nahrungsmittel-, Kosmetik- und Pharmaindustrie von großer Bedeutung ist. Das pufferfreie System bietet außerdem den Vorteil, daß die Produkte keine Reste von Puffersalzen enthalten. Aufwendige Reinigungsschritte zur Entfernung dieser Salze, die bei bestimmten Anwendungen in der Lebensmittel- und Pharmaindustrie stören würden, sind daher nicht erforderlich. Dies führt zu einer weiteren starken Kostenreduzierung. Neben den wasser-unlöslichen α-1 ,4-Glucanen entsteht bei dem erfindungsgemäßen Verfahren Fructose. Diese kann zur kostengünstigen Gewinnung von sogenannten "high-Fructosesyrups" (HFS) verwendet werden. Das erfindungsgemäße Verfahren führt aufgrund der pufferfreien Reaktionsbedingungen zu Produkten hoher Reinheit. Eine aufwendige Reinigung der Fructose ist daher im Gegensatz zu herkömmlichen Verfahren zur HFS-Herstellung aus Maisstärke, die kostenaufwendige Verfahrensschritte zur Entfernung der Puffersalze durch Ionenaustausch umfassen (Crabb and Mitchinson, TIBTECH 15 (1997), 349-352), nicht erforderlich.

Unter einer "in vitro-Umsetzung" wird im Rahmen der vorliegenden Erfindung eine Umsetzung, d.h. eine Reaktion, verstanden, die außerhalb eines lebenden Organismus abläuft. "In vitro" bedeutet insbesondere, daß das erfindungsgemäße Verfahren in einem Reaktionsgefäß stattfindet.

Unter einem Enzym mit der enzymatischen Aktivität einer Amylosaccharase (E.C. 2.4.1.4.) wird ein Enzym verstanden, das die folgende Reaktion katalysiert:

$$\text{Saccharose} + (\alpha\text{-1,4-Glucan})_n \rightarrow \text{Fructose} + (\alpha\text{-1,4-Glucan})_{n+1}$$

Die enzymatische Aktivität einer Amylosaccharase kann z.B. nachgewiesen werden, wie in den Beispielen der vorliegenden Anmeldung beschrieben.

Im Rahmen der vorliegenden Erfindung wird unter einer Amylosaccharase auch ein Enzym verstanden, welches ausgehend von Saccharose und verzweigten Polysaccharidakzeptoren, wie z.B. Glycogen, Amylopektin oder Dextrin, die Synthese von Fructose und von linearen α-1,4-Glucanketten an diesen Polysaccharidakzeptoren katalysiert. D.h. die Amylosaccharase katalysiert eine α-1,4-Glucankettenverlängerung auch an diesen verzweigten Akzeptoren. Die dabei entstehenden Produkte weisen im Vergleich zu den eingesetzten verzweigten Edukten einen geringeren Verzweigungsgrad auf. Auch diese Produkte werden im Rahmen der vorliegenden Erfindung als wasserunlösliche α-1,4-Glucane bezeichnet.

Im Prinzip kann in dem erfindungsgemäßen Verfahren jede beliebige Amylosaccharase eingesetzt werden. Bevorzugt wird eine Amylosaccharase prokaryontischen Ursprungs verwendet. Derartige Enzyme sind beispielsweise bekannt aus Neisseria perflava (Okada und Hehre, J. Biol. Chem. 249 (1974), 126-135; MacKenzie et al., Can. J. Microbiol. 23 (1977), 1303-1307) oder Neisseria canis, Neisseria cinerea, Neisseria denitrificans, Neisseria sicca und Neisseria subflava (MacKenzie et al., Can. J. Microbiol. 24 (1978, 357-362). Weiterhin beschreibt die WO 95/31553 eine Amylosaccharase aus Neisseria polysaccharea. Besonders bevorzugt wird eine natürlicherweise von einem Prokaryonten sekretierte Amylosaccharase verwendet.

In einer bevorzugten Ausführungsform des erfindungsgemäßen Verfahrens wird eine Amylosaccharase von einem Bakterium der Gattung Neisseria verwendet, besonders bevorzugt eine Amylosaccharase von der Species Neisseria polysaccharea.

Wasserunlösliche α-1,4-Glucane im Sinne der Erfindung sind die durch die oben beschriebene Umsetzung von Saccharose mit einer Amylosaccharase hergestellten Polysaccharide. Unter dem Begriff "wasserunlösliche Glucane" versteht man insbesondere die durch die oben beschriebene Umsetzung von Saccharose mit einer Amylosaccharase hergestellten Polysaccharide, die nach der Definition des Deutschen Arzneimittelbuches (DAB = Deutsches Arzneimittelbuch, Wissenschaftliche Verlagsgesellschaft mbH, Stuttgart, Govi-Verlag GmbH, Frankfurt, 9. Auflage, 1987) unter die Kategorie "schwer lösliche" Verbindungen, "sehr schwer lösliche" oder "praktisch unlösliche" Verbindungen fallen.

Unter dem Begriff "pufferfreies System" wird im Rahmen dieser Erfindung ein wäßriges System verstanden, das im wesentlichen keine Puffersalze enthält. Unter dem Begriff "Puffersalze" versteht man in diesem Zusammenhang anorganische und organische Salze, insbesondere Salze schwacher Säuren und Basen.
Unter dem Begriff "im wesentlichen keine" versteht man in diesem Zusammenhang Puffersalzkonzentrationen von höchstens 25 mM, in einer bevorzugten Ausführungsform von höchstens 10 mM, in einer weiteren bevorzugten Ausführungsform von höchstens 5 mM und in einer ganz besonders bevorzugten Ausführungsform von höchstens 1 mM.

In einer weiteren besonders bevorzugten Ausführungsform des erfindungsgemäßen Verfahrens kann ein wäßriges System verwendet werden, welches anorganische und organische Salze als Verunreinigung nur in Spuren (<1 mM) enthält. Ganz besonders bevorzugt ist das wäßrige pufferfreie System reines Wasser.

In einer besonders bevorzugten Ausführungsform des erfindungsgemäßen Verfahrens wird eine gereinigte Amylosaccharase eingesetzt. Unter einer gereinigten Amylosaccharase wird dabei ein Enzym verstanden, das weitgehend frei ist von Zellbestandteilen der Zellen, in denen das Protein synthetisiert wird. Vorzugsweise bedeutet der Begriff "gereinigte Amylosaccharase" eine Amylosaccharase, die einen Reinheitsgrad von mindestens 80 %, bevorzugt von mindestens 90 % und besonders bevorzugt von mindestens 95 % aufweist.

Der Einsatz eines gereinigten Proteins zur Herstellung von $\alpha$-1,4-Glucanen bietet verschiedene Vorteile. Im Vergleich zu Verfahren, die mit partiell aufgereinigten Proteinextrakten arbeiten, enthält das Reaktionsmedium des erfindungsgemäßen Verfahrens keine Reste des Produktionsstammes (Mikroorganismus), der verwendet wird, um das Protein zu reinigen oder gentechnisch herzustellen.

Des weiteren sind durch den Einsatz des gereinigten Proteins, Vorteile für die Anwendung in der Lebensmittel- und Pharmaindustrie zu sehen. Durch die definierte und von allen unnötigen Bestandteilen befreite Zusammensetzung des Reaktionsmediums ist auch das Produkt in seinen Bestandteilen genauer definiert. Dies führt zu einem wesentlich weniger umfangreichen Zulassungsverfahren für diese biotechnologisch erzeugten Produkte in der Lebensmittel- und Pharmaindustrie, insbesondere deshalb, weil diese Produkte keine Spuren eines transgenen Mikroorganismus aufweisen sollten.

In einer besonders bevorzugten Ausführungsform des erfindungsgemäßen Verfahrens ist die Amylosaccharase ein rekombinant hergestelltes Protein. Darunter wird im Rahmen der vorliegenden Erfindung ein Protein verstanden, das dadurch hergestellt wurde, daß eine das Protein codierende DNA-Sequenz in eine Wirtszelle eingebracht und dort zur Expression gebracht wird. Das Protein kann dann anschließend aus der Wirtszelle und/oder aus dem Kulturmedium gewonnen werden. Die Wirtszelle ist dabei vorzugsweise ein Bakterium oder ein Protist (z.B. Pilze, insbesondere Hefen, Algen) wie z. B. definiert in Schlegel "Allgemeine Mikrobiologie" (Georg Thieme Verlag, 1985, 1-2). Besonders bevorzugt wird die Amylosaccharase von der Wirtszelle sekretiert. Die Herstellung derartiger Wirtszellen zur Produktion einer rekombinanten Amylosaccharase kann nach dem Fachmann bekannten Methoden erfolgen.

Eine Übersicht über verschiedene Expressionssysteme findet man z.B. in Methods in Enzymology 153 (1987), 385-516 und in Bitter et al. (Methods in Enzymology 153 (1987), 516-544). Expressionsvektoren sind in großem Umfang in der Literatur beschrieben. Sie enthalten neben einem Selektionsmarkergen und einem die Replikation in dem gewählten Wirt sicherstellenden Replikationsursprung in der Regel einen bakteriellen oder viralen Promotor, sowie meist ein Terminationssignal für die Transkription. Zwischen Promotor und Terminationssignal befinden sich mindestens eine Restriktionsschnittstelle oder ein Polylinker, die die Insertion einer codierenden DNA-Sequenz ermöglichen. Als Promotorsequenz kann, sofern sie in dem gewählten Wirtsorganismus aktiv ist, die natürlicherweise die Transkription des entsprechenden Gens steuernde DNA-Sequenz verwendet werden. Diese Sequenz kann aber auch gegen andere Promotor-Sequenzen ausgetauscht werden. Es können sowohl Promotoren verwendet werden, die eine konstitutive Expression des Gens bewirken, als auch induzierbare Promotoren, die eine gezielte Regulation der Expression des nachgeschalteten Gens erlauben. Bakterielle und virale Promotorsequenzen mit diesen Eigenschaften sind in der Literatur ausführlich beschrieben. Regulatorische Sequenzen zur Expression in Mikroorganismen (z.B. E. coli, S. cerevisiae) sind ausreichend in der Literatur beschrieben. Promotoren, die eine besonders starke Expression des nachgeschalteten Gens erlauben, sind z.B. der T7-Promotor (Studier et al., Methods in Enzymology 185 (1990), 60-89), lacuv5, trp, trp-lacUV5 (DeBoer et al., in Rodriguez and Chamberlin (Eds), Promotors, Structure and Function; Praeger, New York, (1982), 462-481; DeBoer et al, Proc. Natl. Acad. Sci. USA (1983), 21-25), Ip1, rac (Boros et al., Gene 42 (1986), 97-100). In der Regel erreichen die Proteinmengen von der Mitte bis gegen Ende der logarithmischen Phase des Wachstumszyklus der Mikroorganismen ihren Höhepunkt. Zur Synthese von Proteinen werden daher bevorzugt induzierbare Promotoren verwendet. Diese führen oft zu höheren Ausbeuten an Protein als konstitutive Promotoren. Die Verwendung starker konstitutiver Promotoren führt über die ständige Transkription und Translation eines clonierten Gens oft dazu, daß Energie für andere wesentliche Zellfunktionen verlorengeht und dadurch das Zellwachstum verlangsamt wird (Bernard R. Glick/ Jack J. Pasternak, Molekulare Biotechnologie (1995), Spektrum Akademischer Verlag GmbH, Heidelberg Berlin Oxford, S. 342.). Um ein Optimum an Proteinmenge zu erreichen, wird daher oft ein zweistufiges Verfahren angewendet. Zuerst werden die Wirtszellen unter optimalen Bedingungen bis zu einer relativ hohen Zelldichte kultiviert. Im zweiten Schritt wird dann die Transkription je nach Art des eingesetzten Promotors induziert. Besonders geeignet ist in diesem Zusammenhang ein durch Lactose- oder IPTG (=Isopropyl-b-D-thiogalactopyranosid) induzierbarer tac-Promotor (deBoer et al., Proc. Natl. Acad. Sci. USA 80 (1983), 21-25). Terminationssignale für die Transkription sind ebenfalls in der Literatur beschrieben.

Die Transformation der Wirtszelle mit der eine Amylosaccharase codierenden DNA kann in der Regel nach Standardmethoden durchgeführt werden, wie z.B. beschrieben in Sambrook et al. (Molecular Cloning: A Laboratory Course Manual, 2nd edition (1989), Cold Spring Harbor Press, New York). Die Kultivierung der Wirtszelle erfolgt in Nährmedien, die den Bedürfnissen der jeweils verwendeten Wirtszelle entsprechen, insbesondere unter Berücksichtigung von pH-Wert, Temperatur, Salzkonzentration, Belüftung, Antibiotika, Vitaminen, Spurenelementen usw.

Die Reinigung des von den Wirtszellen produzierten Enzyms kann nach herkömmlichen Reinigungsmethoden wie Fällung, Ionenaustausch-Chromatographie, Affinitäts-Chromatographie, Gelfiltration, HPLC Reverse Phase Chromatographie usw. erfolgen.

Durch Modifikation der in den Wirtszellen exprimierten, eine Amylosaccharase codierenden DNA läßt sich in der Wirtszelle ein Polypeptid herstellen, das aufgrund bestimmter Eigenschaften leichter aus dem Kulturmedium isoliert werden kann. So besteht die Möglichkeit, das zu exprimierende Protein als Fusionsprotein mit einer weiteren Polypeptidsequenz zu exprimieren, deren spezifische Bindungseigenschaften die isolierung des Fusionsproteins über Affinitätschromatographie ermöglichen (z.B. Hopp et al., Bio/Technology 6 (1988), 1204-1210; Sassenfeld, Trends Biotechnol. 8 (1990), 88-93).

In einer bevorzugten Ausführungsform des erfindungsgemäßen Verfahrens wird eine Amylosaccharase verwendet, die rekombinant hergestellt ist und von der Wirtszelle in das Nährmedium sekretiert wurde, so daß kein Aufschluß von Zellen und keine weitere Aufreinigung des Proteins erforderlich ist, weil das sekretierte Protein aus dem Überstand gewonnen werden kann. Zur Entfernung von Restbestandteilen des Kulturmediums können in der Verfahrenstechnik gängige Methoden, wie z.B. Dialyse, reverse Osmose, chromatographische Methoden etc. eingesetzt werden. Gleiches gilt auch für die Aufkonzentrierung des in das Kulturmedium sekretierten Proteins. Die Sekretion von Proteinen durch Mikroorganismen wird normalerweise durch N-terminale Signalpeptide (Signalsequenz, leaderpeptid) vermittelt. Proteine mit dieser Signalsequenz können die Zellmembran des Mikroorganismus durchdringen. Eine Sekretion von Proteinen kann dadurch erreicht werden, daß die DNA-Sequenz, die dieses Signalpeptid codiert, an die entsprechende, die Amylosaccharase codierende Region angefügt wird.

Bevorzugt ist das Signalpeptid das natürliche Signalpeptid der exprimierten Amylosaccharase, besonders bevorzugt das der Amylosaccharase aus Neisseria polysaccharea.

Ganz besonders bevorzugt ist das Signalpeptid das der α-CGTase aus Klebsiella oxytoca M5A1 (Fiedler et al., J. Mol. Biol. 256 (1996), 279-291) oder ein Signalpeptid, wie es von den Nucleotiden 11529-11618 der unter der Zugriffsnummer X86014 in der GenBank zugänglichen Sequenz codiert wird.

Alternativ kann die in den erfindungsgemäßen Verfahren verwendete Amylosaccharase auch unter Verwendung eines in vitro-Transkriptions- und Translationssystem, das zur Expression des Proteins führt, ohne Einsatz von Mikroorganismen hergestellt worden sein.

In einer bevorzugten Ausführungsform wird in dem erfindungsgemäßen Verfahren bei der Umsetzung der Saccharose mit der Amylosaccharase ein externer Kohlenhydrat-Akzeptor zugesetzt.

Im Rahmen der vorliegenden Erfindung wird unter einem externen Kohlenhydrat-Akzeptor ein Molekül verstanden, das in der Lage ist, die Anfangsgeschwindigkeit der Umsetzung der Saccharose durch die Amylosaccharase zu erhöhen. Bevorzugt wird der externe Kohlenhydrat-Akzeptor zu Beginn der Umsetzung dem Reaktionsgemisch zugesetzt. Der Einsatz externer Akzeptoren führt zu einer Reduzierung der Verfahrensdauer und damit zu einer Kostensenkung des Verfahrens. Der Kohlenhydrat-Akzeptor ist vorzugsweise ein Oligo- oder Polysaccharid, bevorzugt ein lineares Polysaccharid, und besonders bevorzugt ein verzweigtes Polysaccharid, wie z.B. Dextrin, Glycogen oder Amylopektin. Findet an diesen Akzeptoren eine α-1,4-Glucan-Kettenverlängerung statt, so entstehenden Produkte, die im Vergleich zum verzweigten Edukt einen wesentlich geringeren Verzweigungsgrad aufweisen. Die Stärke der Erniedrigung des Verzweigungsgrades hängt dabei vom Polymerisationsgrad n ab. Setzt man Saccharose in großem molarem Überschuß im Verhältnis zum Akzeptor ein, so sind im Produkt α-1,6-Verzweigungen mittels Methylierungsanalyse nicht mehr meßbar (Verzweigungsgrad < 1 %). Auch diese Produkte werden im Rahmen der vorliegenden Erfindung als wasserunlösliche α-1,4-Glucane bezeichnet.

In einer weiteren bevorzugten Ausführungsform ist das Enzym mit der enzymatischen Aktivität einer Amylosaccharase an einem Trägermaterial immobilisiert.

Eine Immobilisierung der Amylosaccharase bietet den Vorteil, daß das Enzym als Katalysator der Synthesereaktion auf einfache Weise aus dem Reaktionsgemisch wiedergewonnen und mehrfach verwendet werden kann. Da die Aufreinigung von Enzymen in der Regel kosten- und zeitintensiv ist, ermöglicht eine Immobilisierung und Wiederverwertung des Enzyms eine erhebliche Kosteneinsparung. Ein weiterer Vorteil ist der Reinheitsgrad der Reaktionsprodukte, die keine Reste an Protein enthalten.

Für die Immobilisierung von Proteinen stehen eine Vielzahl von Trägermaterialien zur Verfügung, wobei die Kopplung an das Trägermaterial über kovalente oder nicht-kovalente Bindungen erfolgen kann (für eine Übersicht siehe: Methods in Enzymology 135, 136, 137). Weite Verbreitung als Trägermaterial haben z.B. Agarose, Algenat, Cellulose, Polyacrylamid, Silica oder Nylon.

Figur 1 zeigt einen Vergleich der Effizienz der in-vitro-Herstellung wasserunlöslicher α-1,4-Glucane durch eine Amylosaccharase aus Neisseria polysaccharea bei Verwendung unterschiedlicher Puffersalzkonzentrationen. Die Effizienz des Verfahrens wurde anhand der Abnahme der Menge an Saccharose bestimmt.

Die nachfolgenden Beispiele erläutern die Erfindung.

Beispiel 1

Reinigung einer Amylosaccharase

Zur Herstellung einer Amylosaccharase wurden E. coli-Zellen verwendet, die mit einer Amylosaccharase aus Neisseria polysaccharea (s. WO 9531553), transformiert waren. Die DNA stammt aus einer genomischen Bibliothek von N. Polysaccharea.

Eine Über-Nacht-Kultur dieser E. coli-Zellen, die die Amylosaccharase aus Neisseria polysaccharea sekretieren, wurde abzentrifugiert und in ca. $^1/_{20}$ Volumen 50 mM Natriumcitratpuffer (pH 6,5), 10 mM DTT (Dithiothreitol), 1 mM PMSF (Phenylmethylsulfonylfluorid) resuspendiert. Anschließend wurden die Zellen mit einer French-Press bei 16.000 p.s.i. zweimal aufgeschlossen. Danach wurde dem Zell-Extrakt 1 mM $MgCl_2$ zugegeben sowie Benzonase (von Merck; 100,000 Units; 250 Units $\mu l^{-1}$) in einer Endkonzentration von 12,5 Units $ml^{-1}$. Anschließend wurde der Ansatz bei 37°C unter leichtem Rühren mindestens 30 min inkubiert. Der Extrakt wurde mindestens 1,5 Stunden auf Eis stehen gelassen. Anschließend wurde 30 min bei 4°C bei ca. 40 000 g zentrifugiert, bis der Überstand relativ klar war.
Es wurde eine Vorfiltration mit einer PVDF Membrane (Millipore "Durapore", o.ä.) durchgeführt, die einen Porendurchmesser von 0,45 µm besaß. Der Extrakt wurde über Nacht bei 4°C stehen gelassen. Vor Durchführung der HI-(hydrophobic interaction)-Chromatographie wurde der Extrakt mit festem Nacl versetzt, und auf eine Konzentration von 2 M Nacl eingestellt. Anschließend wurde wiederum für 30 min bei 4°C und ca. 40 000 mg zentrifugiert. Danach wurde der Extrakt von letzten Resten an E. coli befreit, indem er mit einer PVDF Membrane (Millipore "Durapore", o.ä.) filtriert wurde, die einen Porendurchmesser von 0,22 µm aufwies. Der filtrierte Extrakt wurde über eine Butylsepharose-4B-Säule (Pharmacia) aufgetrennt (Volumen der Säule: 93 ml, Länge: 17,5 cm). Ca. 50 ml Extrakt mit einer Amylosaccharase-Aktivität von 1 bis 5 Units $\mu l^{-1}$ wurden auf die Säule gegeben. Anschließend wurden mit 150 ml Puffer B nicht-bindende Proteine von der Säule (Puffer B: 50 mM Natriumcitrat pH 6,5, 2 M NaCl) gewaschen. Die Amylosaccharase wurde schließlich mit Hilfe eines fallenden, linearen NaCl-Gradient eluiert (von 2 M bis zu 0 M Nacl in 50 mM Natriumcitrat in einem Volumen von 433 ml bei einer Zuflußrate von 1,5 ml $min^{-1}$ ), welcher mit Hilfe eines automatischen Pumpsystems (FPLC, Pharmacia) generiert wurde. Die Elution der Amylosaccharase erfolgt zwischen 0,7 M und 0,1 M Nacl. Die Fraktionen wurden gesammelt, über eine PD10 Sephadex Säule (Pharmacia) entsalzen, mit 8,7 % Glycerol stabilisiert, auf Amylosaccharase-Aktivität überprüft und schließlich in Lagerpuffer (8,7 % Glycerol, 50 mM Citrat) eingefroren.

Beispiel 2

Bestimmung der Amylosaccharase-Aktivität

Aufgereinigtes Protein oder Proteinrohextrakt wird in unterschiedlichen Verdünnungen in 1 ml Ansätzen enthaltend 5 % Saccharose, 0,1 % Glycogen und 100 mM Citrat pH 6,5 gegeben und bei 37°C inkubiert. Nach 5 min, 10 min, 15 min, 20 min, 25 min und 30 min werden diesem Ansatz je 10 µl entnommen und durch sofortiges Erhitzen auf 95°C wird die enzymatische Aktivität der Amylosaccharase beendet. Im gekoppelten photometrischen Test wird anschließend der Anteil der durch die Amylosaccharase freigesetzten Fructose bestimmt. Dazu werden 1 µl bis 10 µl der inaktivierten Probe in 1 ml 50 mM Imidazolpuffer pH 6,9, 2 mM MgCl2, 1 mM ATP, 0,4 mM NAD und 0,5 U/ml Hexokinase gegeben. Nach sequentieller Zugabe von Glucose-6-phosphat Dehydrogenase (aus Leuconostoc mesenteroides) und Phosphoglucose-Isomerase wird die Absorptionsänderung bei 340 nm gemessen. Anschließend wird mit Hilfe des Lambert-Beerschen-Gesetzes die Menge an freigesetzter Fructose berechnet.
Setzt man den erhaltenen Wert mit dem Zeitpunkt der Probennahme in Beziehung, so läßt sich die Zahl der Units (1U = µmol Fructose/min) (pro µl Proteinextrakt bzw. µg aufgereinigtes Protein) bestimmen.

Beispiel 3

Reaktion im pufferfreien System im Vergleich zum gepufferten System

| | |
|---|---|
| Ansatzvolumina | 50 ml |
| Enzymaktivität | 5 Units / ml |
| Puffer | Na-Acetat pH 6,5, variiert zwischen 0 mM (=Wasser) und 200 mM (Merck) |
| Substrat | 10 % Saccharose (ICN) |

(fortgesetzt)

| | |
|---|---|
| Primer | 0,1 % Dextrin, Typ IV Kartoffel (Sigma) |

Durchführung:

Ansätze von jeweils 50 ml Reaktionsvolumen, enthaltend 10 % Saccharose, 0,1 % Dextrin, 250 Units Amylosucrase und unterschiedliche Konzentrationen eines Reaktionspuffers (25 mM, 50 mM, 100 mM bzw. 200 mM Na-Acetat, pH 6,5) wurden bei 37°C für 46 h bzw. 73,25 h inkubiert. Zusätzlich wurde ein Reaktionsgemisch ohne Puffer, d. h. in demineralisiertem Wasser (pH 7,0) angesetzt. Mit Ausnahme der Puffersubstanz enthielt dieser Reaktionsansatz alle oben aufgeführten Komponenten.

Zur Bestimmung der Umsetzung von Saccharose zu Amylose und Fructose wurden den sechs Reaktionsansätzen zu verschiedenen Zeitpunkten jeweils 1 ml Aliquots entnommen. Die Reaktion wurde in den entnommenen Proben durch 10 minütiges Erhitzen auf 95°C gestoppt. Die Bestimmung der Umsatzraten erfolgte durch Messung der entstandenen Fructose bzw. der Bestimmung der noch vorhandenen Konzentration an Saccharose in den inaktivierten Proben mittels eines gekoppelten enzymatischen Tests im Photometer.

Enzymassay:

| | |
|---|---|
| Meßvolumen | 1 ml |
| Enzyme | Hexokinase aus Hefe, Phosphoglucose-Isomerse, Glucose-6-Phosphatdehydrogenase aus Leuconostoc mesenteroides, β-Fructosidase aus Hefe (alle Enzyme: Boehringer Mannheim) |
| Meßpuffer | 1 mM ATP |
| | 0,4 mM $NAD^+$ |
| | 50 mM Imidazol pH 6,9 |

Der Test beruht auf der Umsetzung von Fructose mittels Hexokinase und Phosphoglucose-Isomerase zu Glucose-6-Phosphat. Anschließend erfolgt die Überführung des Glucose-6-Phosphates mittels Glucose-6-Phosphatdehydrogenase zu 6-Phosphogluconat. Diese Reaktion ist an die Umwandlung von $NAD^+$ zu NADH + $H^+$, die photometrisch bei einer Wellenlänge von 340 nm gemessen werden kann, gebunden. Mit Hilfe des Lambert-Beerschen Gesetzes kann aus den erhaltenen Absorptionen die Menge an Fructose berechnet werden.

Zur Bestimmung der Konzentration von Saccharose wird der zu bestimmenden Probe zusätzlich zu dem oben beschriebenen Reaktionsgemisch noch β-Fructosidase zugesetzt. Diese spaltet die Saccharose in Fructose und Glucose. Die Konzentration beider aus dieser Reaktion entstehender Monosaccharide werden dann wie oben beschrieben mittels der Umwandlung von $NAD^+$ zu NADH + $H^+$ bestimmt. Aus der Summe der bestimmten Monosaccharide kann die Saccharosekonzentration berechnet werden.

Ergebnis:

Nach ca. 73 h ist unter allen Reaktionsbedingungen die im Reaktionsansatz vorhandene Saccharose zu annähernd 100 % zu Amylose und Fructose umgesetzt worden.

**Patentansprüche**

1. Verfahren zur Herstellung wasserunlöslicher α-1,4-Glucane, bei dem Saccharose in vitro durch ein Enzym mit der enzymatischen Aktivität einer Amylosaccharase zu wasserunlöslichen α-1,4-Glucanen und Fructose umgesetzt wird, **dadurch gekennzeichnet, daß** die Umsetzung in einem wäßrigen, pufferfreien System durchgeführt wird.

2. Verfahren nach Anspruch 1, wobei die Amylosaccharase ein Enzym von einem prokaryontischen Organismus ist.

3. Verfahren nach Anspruch 2, wobei der prokaryontische Organismus der Gattung Neisseria angehört.

4. Verfahren nach Anspruch 3, wobei der prokaryontische Organismus Neisseria polysaccharea ist.

**5.** Verfahren nach einem der Ansprüche 1 bis 4, wobei die Amylosaccharase rekombinant hergestellt ist.

**6.** Verfahren nach einem der Ansprüche 1 bis 5, wobei eine gereinigte Amylosaccharase eingesetzt wird.

**7.** Verfahren nach einem der Ansprüche 1 bis 6, wobei die Amylosaccharase an ein Trägermaterial gebunden ist.

**8.** Verfahren nach einem der Ansprüche 1 bis 7, wobei ein externer Kohlenhydrat-Akzeptor zugesetzt wird.

## Claims

**1.** A method for preparing water-insoluble α-1,4-glucans in which sucrose is converted in vitro to water-insoluble α-1,4-glucans and fructose by an enzyme having the enzymatic activity of an amylosucrase, which comprises carrying out the reaction in an aqueous, buffer-free system.

**2.** The method as claimed in claim 1, wherein the amylosucrase is an enzyme from a prokaryotic organism.

**3.** The method as claimed in claim 2, wherein the prokaryotic organism belongs to the genus Neisseria.

**4.** The method as claimed in claim 3, wherein the prokaryotic organism is Neisseria polysaccharea.

**5.** The method as claimed in one of claims 1 to 4, wherein the amylosucrase is produced as a recombinant.

**6.** The method as claimed in one of claims 1 to 5, wherein a purified amylosucrase is used.

**7.** The method as claimed in one of claims 1 to 6, wherein the amylosucrase is bound to a support material.

**8.** The method as claimed in one of claims 1 to 7, wherein an external carbohydrate acceptor is added.

## Revendications

**1.** Procédé de préparation de α-1,4-glucanes insolubles dans l'eau, dans lequel on fait réagir du saccharose *in vitro* à l'aide d'une enzyme présentant l'activité enzymatique d'une amylosaccharase, pour former des α-1,4-glucanes insolubles dans l'eau et du fructose, **caractérisé en ce que** l'on réalise la réaction dans un système aqueux exempt de tampons.

**2.** Procédé selon la revendication 1, dans lequel l'amylosaccharase est une enzyme d'un organisme procaryote.

**3.** Procédé selon la revendication 2, dans lequel l'organisme procaryote appartient au genre *Neisseria.*

**4.** Procédé selon la revendication 3, dans lequel l'organisme procaryote est *Neisseria polysaccharea.*

**5.** Procédé selon l'une quelconque des revendications 1 à 4, dans lequel on produit l'amylosaccharase par recombinaison.

**6.** Procédé selon l'une quelconque des revendications 1 à 5, dans lequel on met en oeuvre une amylosaccharase purifiée.

**7.** Procédé selon l'une quelconque des revendications 1 à 6, dans lequel l'amylosaccharase est liée à une matière support.

**8.** Procédé selon l'une quelconque des revendications 1 à 7, dans lequel on ajoute un accepteur de glucides externe.

Fig. 1

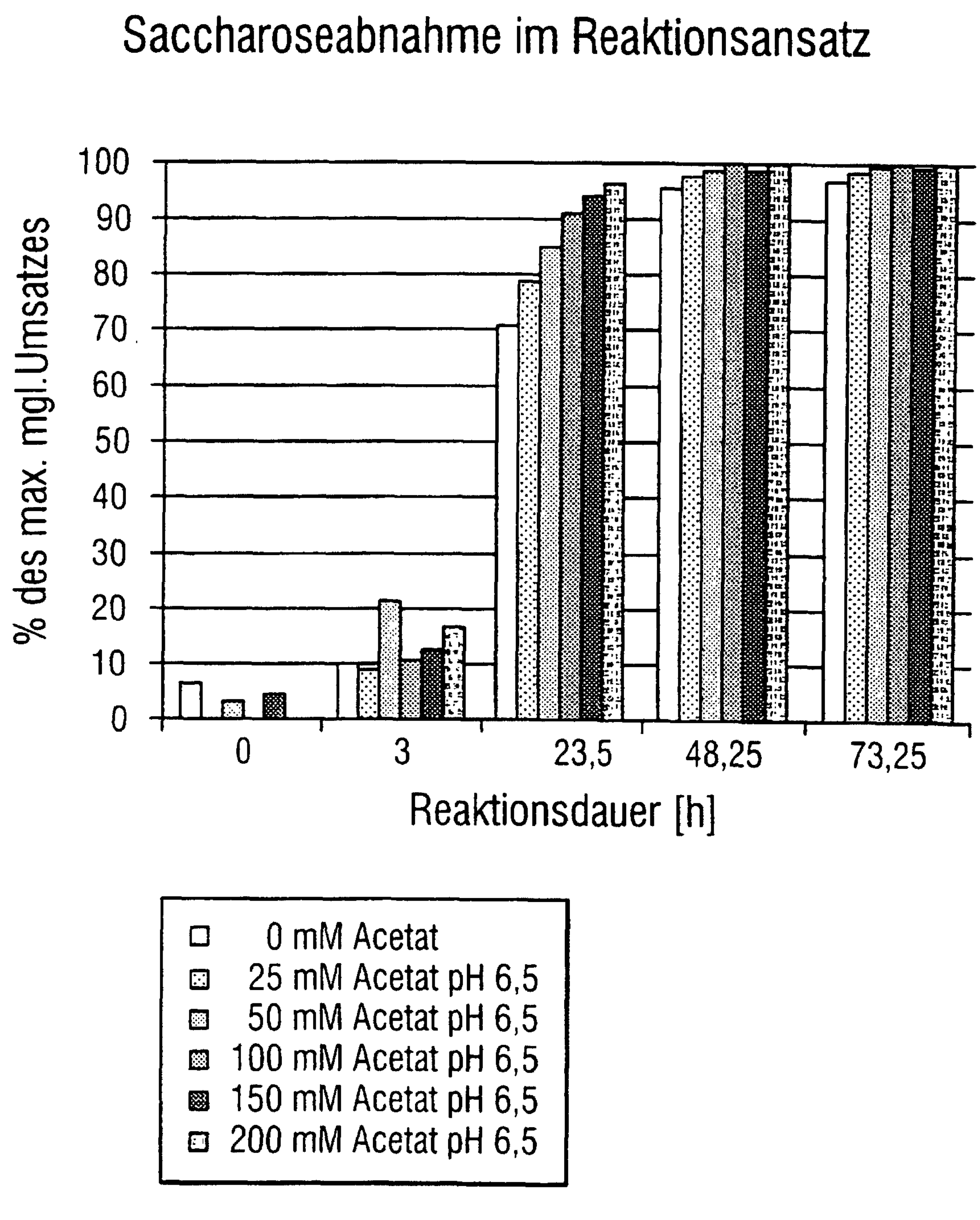
Saccharoseabnahme im Reaktionsansatz
% des max. mgl.Umsatzes
100
90
80
70
60
50
40
30
20
10
0
0
3
23,5
48,25
73,25
Reaktionsdauer [h]
0 mM Acetat
25 mM Acetat pH 6,5
50 mM Acetat pH 6,5
100 mM Acetat pH 6,5
150 mM Acetat pH 6,5
200 mM Acetat pH 6,5